# EUROPEAN PATENT APPLICATION

(11) **EP 1 972 684 A1**
(43) Date of publication of application: **24.09.2008**
(21) Application number: 03730223.9
(22) Date of filing: 11.06.2003
(51) Int. Cl.: C12N 5/02, C12N 5/08, A61K 35/34, A61P 21/00

(54) **MEDIUM FOR CULTURING AUTOLOGOUS HUMAN PROGENITOR STEM CELLS AND APPLICATIONS**

(30) Priority: 02.07.2002 ES 200201540
(71) Applicant: INSTITUTO CIENTIFICO Y TECNOLOGICO DE NAVARRA, S.A., 31008 Pamplona (ES); Chacques, Juan Carlos, 31008 Pamplona (ES)
(72) Inventor: CHACQUES, Juan, Carlos, 31008 Pamplona (ES); PROSPER CARDOSO, Felipe, 31008 Pamplona (ES); HERREROS GONZALEZ, Jesus, 31008 Pamplona (ES)
(74) Representative: Carpintero Lopez, Francisco
(86) International application number: PCT/ES2003/000285
(87) International publication number: WO 2004/005494

(57) **Abstract**

The invention relates to a medium for the autologous culture of autologous human progenitor stem cells, which comprises: between 0.1 and 90 wt.- % autologous human serum; between 0.1 and 10,000 UI/ml heparin; between 0.1 and 10,000 UI/ml protamine; and a culture medium consisting of basic nutrients with or without glutamine, in a sufficient quantity to make up 100 wt.- %, which can be used to culture and expand autologous human progenitor stem cells. Compositions containing said cells can be implanted in the patient using an autologous cellular cardiomyoplasty method in order to create, regenerate and repair dysfunctional myocardial tissue.

## Description

### FIELD OF THE INVENTION

The invention relates, in general, to the obtention and manipulation of autologous human progenitor stem cells under conditions which allow their use in cell therapy. Particularly, the invention refers to a medium for the autologous culture of such cells and to their use in the culture and expansion of the mentioned cells as well as to the compositions which contain them.

### BACKGROUND OF THE INVENTION

One of the biggest challenges for the biomedical research lies in the development of therapeutical strategies which allow to replace or repair cells or tissues damaged or destroyed in the most devastating or disabling diseases: neurodegenerative diseases (Parkinson's, Alzheimer's), diabetes, muscular and cardiac diseases, hepatic insufficiency, etc.

One of the most promising therapeutical strategies is the Cell Therapy, founded in the well-known fact that it is possible to have available cells, more or less undifferentiated, which are able to divide themselves generating functional differentiated cells and, in some cases, can even regenerate themselves. Among these are included the stem cells and the progenitor/precursor cells, which shall be jointly called progenitor stem cells. These cells can be found in the embryo and even in adult tissues. Cell therapy consists, therefore, in the transplantation or implant in the patient of the sufficient quantity of progenitor stem cells to repair and restore the functionality of a damaged organ.

One of the proposed methods of cell therapy is based in the use of progenitor stem cells from an adult, obtained from an animal (xenogenic cells), coming from a donor (allogenic cells), or obtained from the proper patient (autologous cells). The use of autologous progenitor stem cells shall be free from some of the disadvantages of the other methods of cell therapy, such as the lack of donors, need of immunosuppresive treatment to avoid rejection by the patient, as well as the ethical conditionings linked to the use of embryonic cells.

Nevertheless, it is necessary for the cell therapy to come true, to develop a better knowledge of which are the suitable cells in each case and how to identify them, and also to develop procedures for a proper manipulation of progenitor stem cells to be appropriate for their clinical use.

The autologous cell cardiomyoplasty (CMP) is a concrete example od cell therapy for the treatment of cardiac diseases (ischemic heart disease, cardiac insufficiency, etc.). A general updated description can be found in Curr. Control. Trials Cardiovasc Med. 2001; 2:208-210 (D.A. Taylor). Procedures and compositions for the isolation, culture, expansion and use of different progenitor stem cells useful in cell CMP can be found in US5130141, WO/0107568, WO/0194555, WO79854301, US2001/0038837 and US5543318.

### SUMMARY OF THE INVENTION

The invention faces, in general, the problem of providing autologous human progenitor stem cells, suitable for their use in cell therapy, and in particular, with the problem of making an available procedure for the proper manipulation of progenitor stem cells to be appropriate for their clinical use, including specific methods for their obtention, culture, purification and expansion.

The provided solution for this invention is based on the fact that inventors have observed that the use of an autologous culture medium, which comprises autologous human serum and nutrients, together with an anticoagulant, allows the *in vitro* culture, the purification and expansion of autologous human progenitor stem cells, which can be used in the elaboration of pharmaceutical compositions for the treatment of diverse pathologies by means of cell therapy.

The invention is illustrated by the obtention of an autologous culture medium, which comprises nutrients, autologous human serum, heparin and protamine, and his use in the culture *in vitro,* purification and expansion of autologous human muscle progenitor stem cells useful for the elaboration of proper pharmaceutical compositions for the treatment of ischemic cardiomyopathies as well as idiopathic cardiomyopathies by means of autologous cell CMP.

One aspect of this invention is related to a culture medium of autologous human progenitor stem cells which comprises autologous human serum and nutrients, together with an anticoagulant and an agent to reverse anticoagulation.

According to another aspect, the invention is related to a method for the preparation of said culture medium of autologous human progenitor stem cells. In a particular implementation, the autologous human serum present in such culture medium has been obtained by plasmapheresis.

According to another aspect, the invention is related to the use of said medium for the culture *in vitro,* purification and expansion of autologous human progenitor stem cells.

According to another aspect, the invention is related to a method for the obtention of a composition of autologous human progenitor stem cells, which comprises the incubation of said autologous human progenitor stem cells in the mentioned culture and purify the autologous human progenitor stem cells obtained.

According to another aspect, the invention is related to a method for the obtention of a composition of autologous human muscle progenitor stem cells, useful for their use in cell therapy, which comprises the incubation of said autologous human muscle progenitor stem cells in the mentioned culture and the purification of the autologous human muscle progenitor stem cells obtained.

According to another aspect, the invention is related to a composition enriched in autologous human muscle progenitor stem cells.

According to another aspect, the invention is related to a pharmaceutical composition which comprises autologous human muscle progenitor stem cells, together with one or more excipients acceptable from a pharmaceutical point of view.

According to another aspect, the invention is related to a therapeutical method of autologous cell CMP to create, regenerate and repair dysfunctional myocardial tissue by means of the implant of a pharmaceutical composition which comprises autologous human muscle progenitor stem cells, regenerators of cardiac tissue, and *ex vivo* expanded in an autologous culture medium.

### DESCRIPTION OF THE DRAWINGS

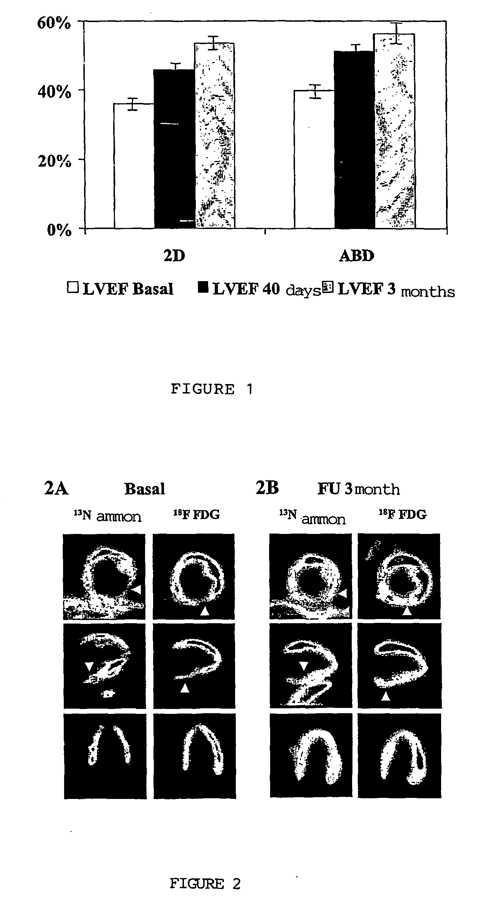

Figure 1 is a bar chart which represents the evaluation of the left ventricular function by analysis of the left ventricular ejection fraction (LVEF), calculated by bidimensional echocardiogram (2D) and authomatic border detection (ABD), before the surgical procedure (Basal), at 40 days of the follow-up visits after the cell transplantation (40 days) and at 3 months follow-up visits (3 months).
Figure 2 shows the evaluation of the perfusion by PET imaging with ¹³N-ammonium and the metabolism of glucose by PET ¹⁸F-FDG, before the surgical procedure (Basal), and at 3 months of the cell transplantation (FU 3 months). Figure 2A: Images corresponding to the patient number 5 as a representative example of a patient which received a cell transplantation. Figure 2B: Images corresponding to the heart of the patient number 9 which do not received a cell transplantation (by contamination of the skeletal myogenic cells culture). The arrows show infarcted tissue, before and after surgery.

### DETAILED DESCRIPTION OF THE INVENTION

### 1. Autologous culture medium

In a first aspect, the present invention is related to an autologous culture medium of autologous human progenitor stem cells, hereinafter referred as "autologous culture medium of the invention", which comprises:
a) between 0,1% and 90% weight of autologous human serum;
b) between 0,1 % and 10,000 UI/ml of heparin;
c) between 0,1 % and 10,000 UI/ml of protamine; and
d) a culture medium with basic nutrients with or without glutamine, in a sufficient quantity up to 100% in weight.

The autologous culture medium of the invention is a complete culture medium processed from autologous human serum obtained from the same patient object of the subsequent implant of the autologous human progenitor stem cells. The autologous human serum, if desired, can be subject to a conventional treatment, for example, thermal treatment, with the aim to inactivate the complement.

The autologous human serum can be obtained from the same patient object of the subsequent implant of the autologous human progenitor stem cells by means of any conventional treatment, for example, from blood samples of the patient or either, preferably, by means of the carrying-out of plasmapheresis to the mentioned patient. In a particular implementation of this invention, the plasmapheresis is done using heparin as anticoagulant and protamine sulphate to reverse anticoagulation. As it is well-known, the plasmapheresis are usually done using ACD (anticoagulant solution acid-citrate-dextrose) [ for example, for 1 litre of water it is added citric acid monohydrate (8g), citrate (22g) and glucose monohydrate (24,5g) as anticoagulant. This anticoagulant provides calcium chelator, then to reverse the effect of the ACD and obtain serum from the plasmapheresis it is necessary to add calcium, which afterwards interferes in the cell culture. The plasmapheresis treatment allows to obtain a significantly high serum supply, superior to the serum obtained from the blood samples of the patient, as in the plasmapheresis there is no blood loss for the patient, which implies an important advantage for the patient. Furthermore, the use of the serum obtained from plasmapheresis with heparin and protamine enables his use in the culture medium without subsequent problems in the cell culture.

The autologous culture medium of the invention contains the basic nutrients and, optionally, glutamine. There exist available commercial mediums which bring the basic nutrients for cell culture. In principle, any medium which provides the adequate nutrients for the cell culture can be used in the present invention. In a particular realization, the nutrients are furnised by the medium HAM F12 [GIBCO BRL].

In a particular realization, the autologous culture medium of the invention contains, besides, an antibiotic. Practically, any antibiotic could be used in the present invention. In a particular realization, the autologous culture medium of the invention comprises an antibiotic selected among penicillin, streptomycin, gentamicin and their mixtures. Likewise, the autologous culture medium of the invention also can contain, if desired, an antimycotic agent, for example, amphoterycin B, and/or a growth factor, for example, a fibroblast growth factor such as the native or recombinant basic Fibroblast Growth Factor (bFGF).

In a particular realization, the autologous culture medium of the invention comprises:
89% weight of medium HAM F12;
10% autologous human serum from the patient;
heparin 0,1- 10,000 UI/ml;
protamine 0,1 to 10,000 UI/ml; and
1% penicillin/streptomycin and, optionally,
0,25 mg/ml amphoterycin B and/or
0,1 to 250 pg/ml recombinant bFGF.

### 2. Method for the preparation of the autologous culture medium of the invention

In another aspect, the invention is related to a method for the preparation of the culture medium of autologous human progenitor stem cells provided by this invention, which comprises to mix and homogenize the different components thereof.

In an advantageous manner, said autologous human serum is obtained by plasmapheresis, as it has been previously mentioned, using heparin as anticoagulant to obtain plasma and protamine to reverse anticoagulation and obtain the serum. In this way, the autologous human serum obtained already contains heparin and protamine.

### 3. Use of the autologous culture medium of the invention

According to another aspect, the invention is related to the use of autologous culture medium of the invention for the culture *in vitro,* purification and expansion of autologous human progenitor stem cells.

The use of autologous human serum in a culture medium for the expansion of autologous human progenitor stem cells presents numerous advantages, for example, cell expansion can be carried out anywhere worldwide, without the risk of contamination by prion disease, viruses and zoonosis inherent to the traditional techniques of cell culture which use fetal bovine serum for cell growth. The use of autologous human serum in human cells cultures instead of bovine serum shows great advantages as (i) it avoids the antigen-antibody reaction, (ii) it avoids inflammatory reactions caused by heterologous proteins; and (iii) it avoids cell destruction as a consequence of the repeated lavages which need the cells cultured in bovine serum before their administration to human beings. In fact, the present clinical experience, with autologous cell CMP, has shown the presence of malignant ventricular arrhythmia in patients 2 weeks after the therapy with bovine serum-cultured cells, complication which, in the 40% of the cases, has required implantable defibrillators.

### 4. Method for the preparation of a composition of autologous human progenitor stem cells

According to another aspect, the invention is related to a method for the preparation of a composition of autologous human progenitor stem cells, which includes to incubate of said autologous human progenitor stem cells in the autologous culture medium of the invention and purify the autologous human progenitor stem cells obtained.

Practically any autologous human progenitor stem cell can be cultured and expanded in the autologous culture medium of the invention, adapting in each case the conditions of the medium and culture to the concrete cell type. Nevertheless, in a particular realization, that shall be described in detail in the next section, such autologous human progenitor stem cells are autologous human muscle progenitor stem cells.

The autologous human progenitor stem cells can be obtained by means of a biopsy in the proper patient object of the subsequent implant of such autologous human progenitor stem cells.

The incubation of the autologous human progenitor stem cells in the autologous culture medium of the invention is carried out under conditions which allow cell growth and division.

The purification of the autologous human progenitor stem cells obtained can be achieved by any conventional method. In a particular realization, The purification of said autologous human progenitor stem cells consists of an immunopurification and it is carried out by the use of specific and selective antibodies for such autologous human progenitor stem cells, which allow the identification of extracellular antigens characteristic of the mentioned autologous human progenitor stem cells. Advantageously, said specific and selective antibodies for such autologous human progenitor stem cells are joined to magnetic microspheres to purify and enrich the autologous human progenitor stem cells by means of a magnetic cell separator.

The autologous human progenitor stem cells obtained by the previously described method, can be presented as pharmaceutical compositions, and can be used in gene therapy.

### 5. Method for the obtention of autologous human muscle progenitor stem cells

As it has been previously mentioned, in a particular and preferred realization of this invention, the autologous human progenitor stem cells are autologous human muscle (or myogenic cells) progenitor stem cells.

Therefore, in another aspect, the invention is related to a method for the obtention of autologous human muscle progenitor stem cells, suitable for their use in cell therapy, which comprises to incubate said autologous human muscle progenitor stem cells in the mentioned culture medium and purify the autologous human muscle progenitor stem cells obtained.

The autologous human muscle progenitor stem cells can be obtained by the carrying out of a biopsy in the proper patient object of the subsequent implant of such autologous human progenitor stem cells, in particular, a skeletal muscle biopsy.

Previous studies state that the administration to a patient, prior to the realization of the biopsy, in the biopsy area, of a pharmaceutical composition which includes a pharmaceutical agent that stimulates the proliferation of autologous human muscle progenitor stem cells allows to increase the number of said cells at the initiation of the culture. In a particular realization, this preconditioning is made through the administration to a patient, before carrying out the skeletal muscle biopsy, usually in a period of time comprised between 2 days and 15 minutes before the fulfillment of the biopsy, via intramuscular, in the area where the biopsy is being carried out, of a pharmaceutical composition which includes said pharmaceutical agent that stimulates cell proliferation. In a particular realization, said pharmaceutical agent is a local analgesic, such as lidocaine or bupivacaine, that stimulate the proliferation of myoblasts, what is later reflected in a higher efficiency of the cell culture.

By the skeletal muscle biopsy a sample of muscle tissue is collected, which is subject to a conventional treatment to digest the muscle fibers as it is later described in more detail. The resulting cell suspension is cultured in the autologous culture medium of the invention. In general, the sample extracted by means of the muscle biopsy contains different types of tissues, such as adipocytes, fibroblasts, mesenquimal progenitors, hematopietic cells, muscle fibers, vascular tissue (endothelium, smooth muscle) and, obviously, myoblasts and satellite cells. Typically, the initial content of autologous human muscle progenitor stem cells present in the sample of skeletal muscle extracted by the biopsy, defined as CD56+/ CD45- is inferior to 10%. Likewise, there also exists a small percentage of stem cells (satellite cells) of very difficult identification which are the ones that reproduce themselves and differ towards muscle cells which express antigen CD56 and which would be muscle progenitors. As it is known, the differentiation process of this cell type comprises the step from satellite cell to myoblast, then to immature myocyte, later to mature myocyte, and finally to myofiber. The muscle progenitors would be the myoblasts and myocytes (mature and immature), which could be identified by the presence of the antigen CD56, the intracytoplasmic expression of desmine and the absence of the antigen CD45 (that is, they present a phenotype CD56+/desmine+/ CD45-). During the cell culture there is produced a proliferation of satellite cells and myoblasts which are changing to, so far, myocytes. Therefore, after incubation and cell expansion, in the culture medium there are accumulating myoblasts and myocytes (mature and immature) that are used in the elaboration of a pharmaceutical composition to inject autologous human muscle progenitor stem cells in the patient and which, once injected, finish their differentiation process in the proper patient becoming muscle fibers or myofibers and developing their function.

The incubation of autologous human muscle progenitor stem cells in the autologous culture medium of the invention is made under conditions which allow cell expansion (growth and division).

The purification of the autologous human muscle progenitor stem cells obtained can be carried out by any conventional method. In a particular realization, the purification of said autologous human muscle progenitor stem cells comprises the use of specific and selective antibodies for these autologous human muscle progenitor stem cells, which allow the identification of extracellular antigens characteristic of said autologous human muscle progenitor stem cells, for example, antibodies human anti- CD56 (CD56 is an extracellular antigen characteristic of skeletal muscle) in absence of the specific antigen of hematopoietic cells CD45 (that is to say, cells which show the phenotype CD56+/ CD45- are selected).

Advantageously, said antibodies are joined to magnetic microspheres to purify and enrich the autologous human muscle progenitor stem cells by means of a magnetic cell separator. In another particular realization, the cell culture is subject to a prior stage of pre-plating with the aim to settle the fibroblasts before proceeding to identify and separate the autologous human muscle progenitor stem cells. In this case, the purification of the autologous human muscle progenitor stem cells comprises to subject the cell culture to a pre-plate stage with the aim to settle all or part of the fibroblasts present in said cell culture and, afterwards, to identify and separate the autologous human muscle progenitor stem cells by the use of antibodies human anti-CD56, in an optional manner, joined to magnetic microspheres, and the selection of cells which show a phenotype CD56+/ CD45-.

The composition which comprises autologous human muscle progenitor stem cells obtained according to the aforementioned method constitutes an additional aspect of this invention.

Alternatively, the invention provides a procedure for the obtention of autologous human muscle progenitor stem cells, starting from a skeletal muscle tissue biopsy, for the preparation of a pharmaceutical composition, which comprises:
a) the fulfillment of a biopsy in a patient object of the subsequent implant of the autologous human muscle progenitor stem cells to extract a fragment of skeletal muscle tissue which comprises autologous human muscle progenitor stem cells;
b) the culture of said autologous human muscle progenitor stem cells coming from the skeletal muscle in the autologous culture medium of the invention, under conditions which allow the expansion of said cultured autologous human muscle progenitor stem cells;
c) the purification of said cultured autologous human muscle progenitor stem cells; and
d) the collection of said purified autologous human muscle progenitor stem cells; and optionally,
e) the freezing of said purified autologous human muscle progenitor stem cells until the preparation of such pharmaceutical composition.

As it has been previously mentioned, the local administration to a patient, in a period of time between 2 days and 15 minutes before the fulfillment of the biopsy, in the area where the biopsy is being carried out, of lidocaine or bupivacaine stimulates the proliferation of the autologous human muscle progenitor stem cells and allows to increase the number of such cells at the initiation of the culture.
The culture of said autologous human muscle progenitor stem cells coming from the skeletal muscle in the autologous culture medium of the invention, under conditions which allow the expansion of said cultured autologous human muscle progenitor stem cells, includes the carrying out of a series of prior conventional treatments, for example, lavage, removal of fatty tissue, muscle crumbling, enzymatic dissociation, filtration and collection of cells by, for example, sedimentation. In general, after the enzymatic digestion of the muscle fibers there is produced a cell suspension which contains a great quantity of cellular detritus. After the culture stages cells and fibers debris are removed and this suspension is enriched in muscle progenitor stem cells.

The purification of the cultured autologous human muscle progenitor stem cells pursues, among others, the aim of obtaining compositions enriched in autologous human muscle progenitor stem cells substantially free of fibroblasts. For this purpose, the cell culture can be subject to a first stage of pre-plating, by which the fibroblasts are sedimented quicker than myoblasts or satellite cells, and to a later process of identification and separation of said autologous human muscle progenitor stem cells by any conventional method, for example, by the use of specific and selective antibodies for such cells, which allow the identification of extracellular antigens characteristic of the mentioned autologous human muscle progenitor stem cells which show a phenotype CD56+/ CD45-, through antibodies that specifically recognize such autologous human muscle progenitor stem cells, joined in an optional way to magnetic microspheres.

According to another aspect, the invention is related to a pharmaceutical composition which comprises autologous human muscle progenitor stem cells, together with, at least, one excipient acceptable from a pharmaceutical point of view. In a particular realization, such pharmaceutical composition is suitable to be administered via parenteral. In principle, any excipient acceptable from a pharmaceutical point of view able to convey the autologous human muscle progenitor stem cells can be used in the current invention. In a particular realization, such pharmaceutical composition includes albumin which is used as excipient to resuspend the autologous human muscle progenitor stem cells.

As it is used in the present invention, the term "pharmaceutical composition" refers to a composition of autologous human muscle progenitor stem cells prepared for its therapeutic use in a cellular implant, which comprises, at least, 20 million cells, with a cellular density of , at least, 50 million cells/ml and, at least, 40% autologous progenitor stem cells CD56+/ CD45-, autologous culture medium of the invention, and at least, one excipient acceptable from a pharmaceutical point of view. In a particular realization, such pharmaceutical composition comprises between 20 and 200 million cells, with a cellular density between 50 and 70 million cells/ml, and at least, 70% autologous progenitor stem cells CD56+/ CD45-, autologous culture medium of the invention, and human albumin in a quantity between 0,1% and 20% weight with respect to the total.

The autologous human muscle progenitor stem cells obtained according to the previously described method, as well as the pharmaceutical compositions which contain them, can be used in the elaboration of a pharmaceutical composition:
for repairing and/or reconstituting cardiac muscle tissue, and/or
for the treatment of post-ischemic cardiac insufficiency, and/or
for the treatment of dilated cardiomyopathy and/or
for the treatment of non-ischemic cardiomyopathy

### 6. Therapeutic procedure

According to another aspect, the invention is related to a therapeutic procedure of autologous cellular CMP to create, regenerate and repair dysfunctional myocardial tissue by means of a pharmaceutical composition implant which includes autologous human muscle progenitor stem cells, due to its capacity to regenerate cardiac tissue, expanded and kept *ex vivo* in an autologous culture medium; said procedure comprising the collection of a material sample coming from a patient object of the subsequent implant which comprises autologous human muscle progenitor stem cells, the expansion of such cells by means of a culture in an autologous culture medium provided by this invention and the implantation of autologous human progenitor stem cells collected from the patient, who previously had been extracted said material containing the autologous human muscle progenitor stem cells.

From a concrete perspective, the invention provides a therapeutic procedure of autologous cellular CMP to create, regenerate and repair dysfunctional myocardial tissue by means of a pharmaceutical composition implant which includes autologous human muscle progenitor stem cells, regenerators of cardiac tissue, and kept *ex vivo* expanded in an autologous culture medium; and where said procedure comprises the following steps:
a) a biopsy of skeletal muscle to the patient taken from a muscle, preferably, preconditioned by an intramuscular injection of a local anesthetic, such as lidocaine or bupivacaine;
b) the preparation of a culture medium of autologous human progenitor stem cells starting from autologous serum of the patient;
c) the preparation of a composition enriched in autologous human muscle progenitor stem cells starting from the biopsy of a) and the culture medium of b);
d) the preparation of a pharmaceutical composition starting from the composition of c); and
e) the implant of a pharmaceutical composition of autologous progenitor stem cells of d) in myocardial lesions.

The autologous human muscle progenitor stem cells comprise myoblasts and myocytes (mature and immature), and have the capacity to regenerate cardiac tissue when they are implanted in the cardiac muscle.

As it is used in the present description, the term "myocardial lesions" refers to ischemic as well as idiopathic cardiomyopathies. In a particular application, the procedure of autologuos cellular CMP according to the present invention is applied to patients with an ischemic cardiomyopathy due to myocardial infarction without possibility of surgical or percutaneous revascularization as well as patients of alternative cardiac revascularization. The aim of this autologuos cellular CMP would be to limit the expansion of the infarction, the cardiac tissue remodelation and the myocardial regeneration. The definition of ischemic cardiomyopathy includes patients with right or left ventricle infarction, in this last case with possibility of ischemic regurgitation of the mitral valve. In another particular application, the autologuos cellular CMP procedure according to the present invention is applied to patients with an idiopathic dilated cardiomyopathy.

Among the advantages of the cellular CMP it can be cited the decrease of fibrosis, the reduction of the scar area of the infarction and the improvement of elasticity and properties of the ventricular wall (essential to restore the diastolic function).

In a particular realization, the implant of a pharmaceutical composition of autologous progenitor stem cells of d) in myocardial lesions is performed by direct injection. The term "implant by direct injection" as it is used in the present invention alludes to an epicardial or endovascular administration of the pharmaceutical composition provided by the present invention in patients with ischemic or idiopathic cardiomyopathies. In a particular realization, it is performed an epicardial or endovascular administration of the pharmaceutical composition of the invention in patients with ischemic cardiomyopathy distributed according to the following percentage:
Approximately 70% in the periferic area of the infarction and 30% approximately in the central part of the scar. The epicardial administration can be done either by conventional surgical exposure or either by thoracoscopy. In the surgical approximation (classic or mini thoraco/sternotomy) the ischemic area becomes well exposed allowing the carrying out of the injections of the therapeutical composition in the infarcted area and, predominantly, in the surrounding zone. The injection of the pharmaceutical composition containing autologous human muscle progenitor stem cells provided in this invention can be also performed in the perilesional area. With this distribution of the implant supported in the present invention it is obtained a major survival of the implanted cells in the peripheral region by residual irrigation and the existing collateral myocardial revascularization and it is avoided to some extent the high cellular mortality observed in the implants which are made in the highly fibrotic ischemic scar.

In another particular realization, the implant of the pharmaceutical composition of autologous progenitor stem cells of d) in myocardial lesions is made by systemic or intracoronary administration through a percutaneous venous access.

In another particular realization, the autologous cellular CMP procedure according to the present invention is applied to patients with an idiopathic dilated cardiomyopathy carrying out multiple implants of the pharmaceutical composition provided by the invention among the coronary arteries in the myocardium of both ventricles.

Nowadays we are provided with new computerized surgical systems for their use in cardiac surgery. The operations of robotic-assisted coronary bridges or bypasses by means of thoracic access is increasing as it permits a quick and safe recovery of the patient with a reduced risk of infections and an excellent cosmetic result. In a particular realization, the needle is inserted in the myocardium by a video-controlled robot arm, the syringe is located outside the torax and is connected with the needle by an expander tube of small diameter. The advantage of this approximation in patients with cardiac insufficiency is that the therapeutic procedure of autologous cellular CMP can be made safely without heart manipulations, avoiding the risk of hemodynamic alteration and ventricular fibrillation.

The beneficial effect of the cellular CMP could be limited by the mortality rate of the injected cells. To solve this problem, the invention proposes to make periodical injections by procedures of percutaneous or surgical implant of the autologous progenitor stem cells of the present invention. This approximation would achieve the aim of the CMP, progressively reducing the infarcted areas or ameliorating the pathologic myocardium. With this aim the invention includes the creation of a customized bank of each autologous progenitor stem cells patient, originated from isolated and frozen cells during the cell culture procedure of each individual. From the stored autologous progenitor stem cells new cell cultures can be expanded for the preparation of new compositions avoiding new biopsies or tissue elimination.

The following examples serve to illustrate the invention and are construed for non-limitative purposes thereof.

### EXAMPLE 1

In this example it is described the obtention and manipulation of autologous human muscle progenitor stem cells for their use in the autologous cellular CMP procedure with the aim to reverse the damaged dysfunctional myocardial tissue in an alive hystological structure able to generate systolic and dyastolic pressure.

### 1.1 Skeletal muscle biopsy

One day before proceeding with the surgical biopsy it was injected to the patient, via intramuscular, a lidocaine solution at 2%, in the anterolateral thight muscle and surroundings. The biopsy is made by a 5 cm incision in the anterolateral thight muscle and, in sterile conditions, a fragment of 2 to 3m³ of skeletal muscle is inserted (15 g). Immediately after the extracted muscle tissue is fragmented with scissors, is inserted in complete culture medium or in a phosphate buffer solution (PBS) and is kept at 4° C. The procedure for the cellular isolation and purification and its culture shall be started as soon as possible to guarantee cell survival. The samples are carried out to the Cellular Biology laboratory in a suitable container and at low temperature.

### 1.2 Preparation of the autologous culture medium

### From blood samples

In a Blood Transfusion Service, venous blood is drawn from the patient. By means of venipuncture 50 tubes of 10ml are filled up for serum collection. After the cellular sedimentation and fibrin precipitation, under laminar flow conditions cabin, the serum is extracted from each tube and collected in an empty blood bag. Serum samples are taken for hematologic and microbiologic trials (bacteria and virus). The bag with the serum is frozen at -80°C waiting for the results of the hematologic and microbiologic trials.

Before preparing the culture medium, the serum complement is heat-inactivated at 56°C for 1 hour and it is filtered. Part of the serum is combined with the culture medium and kept at 4°C, then heated up to 37°C and filtered before adding the bottle with the cell culture. The final culture medium contains 10% of autologous human serum of the patient, 89% of medium HAM-F12 (GIBCO-BRL , Cat. No. 21765), 1% penicillin/streptomycin, heparin (2UI/ml of collected serum), protamine (in sulphate), (3UI per ml of collected serum), and optionally, amphoterycin B (0,25 mg/ml) and/or 0,1 to 250 pg/ml of recombinant bFGF.

The remaining human serum is frozen at -40°C waiting for a new culture medium preparation. In every culture preparation the complement is again inactivated at 56°C for 1 hour with a later filtration.

### From plasmapheresis

Immediately after initiating the plasmapheresis procedure the patient is administered a 50 UI/kg dose of non-fractioned sodium heparin. The process of plasmapheresis is carried out according to the expected routine procedure of the equipment used. During plasmapheresis the standard anticoagulant is substituted by a physiological solution of sodium chloride 0,9% with heparin as anticoagulant. The plasma is substituted by albumin at 5%. The procedure is finished once obtained the necessary volume of plasma for the preparation of the autologous culture medium. The quantity of heparin in the collected plasma is calculated according to the plasmatic volume of the patient and it is added 1,5UI of protamine of each UI of heparin. It is kept like that until there is produced the plasma coagulation. Then serum is extracted and distributed in small aliquots which are forzen. The same with the serum collected from blood samples, also in this case the serum samples were hematologically and microbiologically analyzed before being used.

### 1.3 Isolation of the satellite cells and expansion in vitro

All the manipulations are carried out in aseptic conditions and using a laminar flow cabin. The fragments of explanted skeletal muscle are washed in PBS. Adipose tissue is removed with scissors and carefully the muscle is crumbled. The muscle fragments are again washed in PBS as the supernatant fraction appears clean. It is centrifuged for 5 minutes at 100g. The tissue is dissociated by 2 consecutive enzimatic treatments: firsly, cells are incubated in collagenase IA (1,5 ml/mg/g tissue) and they are kept incubating for 1 hour. Every ten minutes the tubes are agitated to mechanically favour the dissociation. Alternatively, the tubes are located in an incubator with reciprocating/orbital agitation (ROSI) at 37°C. Secondly, it is incubated for 20 minutes with 0,25 trypsin 1 X EDTA (2ml). Immediately after, cells are washed (10 minutes at 300 g) and to stop enzimatic reaction it is added 1ml of the proper serum of the patient previously obtained. A filtration is fulfilled through a net of 40 µm (cell stainer nylon). With the fragments which could have been left in the net, the digestion procedure is repeated again. Finally the filtration cells are collected by sedimentation (20 minutes at 300g) and the supernatant is rejected.
Cells are resuspended in a fresh complete culture medium: 10% autologous human serum of the patient, 89% of medium HAM-F12 (GIBCO-BRL , Cat. No. 21765), 1% penicillin/streptomycin, and optionally, amphoterycin B (0,25 mg/ml) which contains, furthermore, heparin (2UI/ml of collected serum), and protamine (in sulphate), (3UI per ml of collected serum), and are seeded in bottles for cell culture. Then, the bottles are incubated for 3 weeks at 37°C in a humid atmosphere and at 5% H²O. The bottles are located in the incubator without level gradient to avoid an irregular cell proliferation. After an incubation period of 2 to 3 days, it is renewed in the medium to remove blood cells and dead cells. When they have grown to subconfluence (50% confluence) one of the step of the cultures are taken (fraction 1:5) to avoid the appearance of myogenic differentiation at high densities. At 50% confluences multiple steps shall be required to prevent that the mononucleated cells differ in multinucleated myotubes.

In each step of the cultures, cells are harvested by trypsination (2 ml of 2,5 trypsine/EDTA in each bottle during 1 to 5 minutes in the incubator). The complete detachment of cells is verified observing through the microscope the floating cells. Then reaction is stopped by the addition of complete culture medium and the suspension of the resulting cells is distributed in other 5 bottles. In each step of the cell culture process there are carried out controls for bacteria (aerobic and anaerobic bacteria trials), viruses and fungus.

### 1.4 Removal of fibroblasts

Usually fibroblasts contaminate cultures, then to eliminate them it is necessary to obtain a suitable myoblasts expansion. In the carrying out of the first pass and, after the neutralization with trypsin, the cultured cells are kept in the incubator for 30 minutes. This period of time is enough for the fibroblasts to sediment while the majority of the myoblasts (smaller) are kept in suspension. The cellular supernatant is collected and is transferred to new culture bottles. The myoblasts purity is measured by flow cytrometry with a human antibody anti-CD56 and intracellular desmin staining. The samples with a myoblasts purity inferior to 50% are subject to a procedure of cellular enrichment. For that purpose, in the second stage, after harvesting cells and before seeding them, those are stained with a mouse antibody human anti-CD56, to which have been joined magnetic microspheres. By means of a positive selection made with a magnetic cell separator there are obtained cellular populations highly enriched in myoblasts, progenitor muscle cells, which express CD56 and desmine in more than 90% of the cells.

Usually, to obtain the final necessary quantity of cells, several passes have to be made. In general, at the end of approximately 3 weeks, there are obtained more than 200 million cells. This number can be escalated by passes in a multichain culture system.

### 1.5 Isolation of cells for the customized bank

During the cell culture process some cells which would be frozen can be isolated for each patient. Starting from these stored cells, new cell cultures can be expanded, to make periodical repeated intramyocardial injections (avoiding then the repetition of new biopsies). In the moment of the second pass, some samples of highly enriched positive cells for CD56 are cryopreserved using 5% DMSO, are subject to a programmed freezing and finally they are stored in liquid nitrogen. Cell concentration shall be 25 to 50 million cells for ml. When necessary, cells are defrosted and cultured in myoblasts medium for their later use (percutaneous implant) once it has been made the *ex vivo* expansion.

### 1.6 Injection medium

The day of the cell implant, cells are harvested and washed in injection medium (human albumin 0,5% and complete culture medium) and are kept on ice before the implant. By means of flow cytometry it is valued the final purity rate of the myoblasts. Using a Malassez cytometer (by trypan blue staining) it is determined cell concentration and viability. Likewise, before its implant, it is valued the cell culture sterility (Gram test).

### 1.7 Cell culture procedure

The cell implant can be performed by an epicardial or endovascular administration. The epicardial administration can be made either by conventional surgical exposure or either by thoracoscopy. In the surgical approximation (classic or mini thoraco/sternotomy) the ischemic area becomes well exposed allowing the achievement of approximately 10 injections of cell suspension in the infarcted area and, predominantly, in the surrounding zone. For this purpose, it is used a curved needle of 23 to 26 G x 4 cm. The recommended cell density is comprised between 50 and 70 million cells/ml. The injection is made slowly, for approximately 15 minutes. After each injection, the orifices of the needle should be blocked by print pressure (1 to 2 minutes) to avoid a regurgitation of the cell suspension.

Implant protocol of the myoblasts:
- implant of, at least, 20 million cells, preferably, about 200 million cells.
- Cell density: 50 to 70 million cells per ml.
- Culture time: approximately 21 days.
- Myoblasts concentration: superior to 70%
- Mean cell life at 2-8°C: 96 hours.

### EXAMPLE 2

This example is a clinical trial in phase I/II carried out to value the suitability and security of the intramyocardial transplantation of a composition of autologous human muscle progenitor stem cells of the invention to patients who have suffered from myocardial infarction.

### MATERIAL AND METHODS

### 2.1 Selection of patients

The trial was carried out over a total number of 12 patients who have previously suffered from a myocardial infarction,at least 4 weeks prior to their inclusion in the protocol, and who were expecting to be subject to an aortocoronary bypass. For the selection of patients it was taken into account age (comprised in the range 30-80 years), cardiac function ( left ventricular ejection fraction superior to 25%) and not having malignant arrhythmias or muscular dystrophy history, nor having shown a positive result in tests of hepatic or nephritic dysfunction or in pregnancy tests, HIV or hepatitis.

### 2.2 Muscular biopsy, culture and characterization of the autologous human muscle progenitor stem cells

The preparation of the autologous human muscle progenitor stem cells for the therapeutical composition was performed such as it is described in the Example 1.

The muscular biopsies were collected from the anterolateral thight muscle under sterile conditions and after a preaconditioning of lidocaine hydrochloride. In the whole procedure it was used an autologous human culture medium of the invention which comprised 79% medium HAM F12 (GIBCO-BRL). The autologous serum of the patient was obtained by plasmapheresis, as it is described in the paragraph 1.2 of the last example, carried out the previous day to the obtention of the muscular biopsy (between 800 and 2,135 ml per patient, average: 1,735 ml).

For the isolation of satellite cells, the enzimatic digestions were carried out by incubation with trypsine/EDTA (0,5 mg/ml and 0,53 mm EDTA, GIBCO-BRL) and subsequently with collagenase (0,5 mg/ml GIBCO-BRL). The procedure of cell expansion and removal of fibroblasts was performed as it has been described in the previous example. The purity of the myobasts cells in the harvested was measured by flow cytometry and staining with specific monoclonal antibodies for human N-CAM (CD56), CD45 and desmin.

The average myogenic cells obtained per patient was 221 x 10⁶ (always between the range 105-390 x 10⁶) and the mean purity of the myogenic cells CD56+/ CD45- was 65,6±6,4%.

### 2.3 Cellular transplantation

Before the surgical procedure, each patient was determined the function and viability of the cardiac muscle through positron emission tomography (PET), echocardiogram and electrocardiogram (Holter EGC 24 hours). The aortocoronary bypass with extracorporeal circulation was carried out between 3 to 4 weeks after having performed the muscular biopsy. The patients received a mean of 2 grafts (between 1 and 4).

Once sutured all the grafts and reestablished the spontaneous heart beat the cellular implant was carried out by epicardic administration by at least 10 repeated injections in the infarcted area and surrounding zones, zones which have been from the echocardiographical point of view have been identified as hypokinetic, akinetic or dyskinetic. For the injections, it was used an ophthalmic cannula 23G (Maersk Medical Ltd. Redditch, B98 9NL GB). The zones which were to receive the cellular implant were identified by echocardiogram before surgical procedure with the aim to evaluate the contractibility of said zones during the later surveillance to the cellular transplantation.

After surgical procedure, the patients were subject to a continuous telemetry monitoring. Blood samples were taken each six hours to evaluate the presence of cardiac necrotic enzymes. To prevent inflamation methylprednosolone was administered (500 mg) after surgery. To prevent cardiac arrythmias, it was reported a treatment of 3 months with oral amiodarone. For the response monitoring to the cardiomyoplasty procedure carried out it was made a follow-up by PET (3 months after transplantation), echocardiogram (40 days and 3 months after transplantation),. Likewise, it was monitored the presence of arrythmias by means of Holter-ECG (40 days and 3 months after transplantation).

The protocol and all the procedures carried out were previously approved by the institutional and regional ethical commitees of clinical trials according to the legally expected requirements.

Echocardiographic researches.The global and regional myocardial contractility was measured by bidimensional echocardiography (by an ultrasounds system Sonos 5500, Phillips) . The analysis of the movement of the regional left ventricular wall was performed according to the procedure described in the Standards Committee of the Amercan Society of Echocardiography (Schiller NB et al, J AM Soc Echocardiogr. 1989; 2:358-367): the left ventriculus was divided in 16 fragments and it was valued for each segment the movement of the wall as 1=normal, 2= hypokinesia, 3=akinesia, 4=dyskinesia. The regional motility index (wall motion score index, WMSI) was determined as the add of each segment indexes divided by the number of the evaluated segments. An index WMSI was obtained for the segments treated with cell implant and other for those non-treated. The left ventricular ejection fraction (LVEF) was obtained by a system of automatic detection of the endocardial border (automatic border detection ABD) (Perez JE et al, J. Am. Coll. Cardiol. 1992; 19:336-344). It was also valued the regional contractility of each segment by colorkinesis and tissue doppler. The evaluations were performed by a double-blind essay (2 independent observers without prior information). The reproducibility in the trial for the final diastolic volume in the left ventriculus was 2,8±6,4 (CV 5,5%), and for the ejection fraction (LVEF) 0,3±4,6 (CV 6,6%).

Positron emission tomography imaging (PET). It was evaluated by PET the myocardial blood flow, and the metabolism glucose, before surgical treatment and at 3 months of the cell transplantation.

Perfusion and metabolism trials were carried out in a PET tomograph (ECAT EXACT HR+, Siemens/CTI knoxville, USA) which acquires 62 transaxial views with a spatial resolution between planes of 4,5 mm. The production of tracers, for the studies of glucose metabolism (¹⁸F-FDG) and for those of perfusion (¹³N-ammonium), it was performed in the cyclotron (cyclone 18/9, Ion Beam Applications, Belgium), and in the radiopharmacy lab of the center.

The protocol of image acquisition in each patient was initiated with 2 minutes-transmission trial using sources of germanium-68 to localise the heart in the field of vision, followed by 5 minutes-acquisition to effectuate correction of photonic attenuation. Immediately after, ¹³N-ammonium was perfused (9,25 MBq/Kg, maximum 740 MBq) by intravenous injection at constant flow of 10ml/min. Dynamic acquisition of images started at the moment of the injection. Serial images in dinamic sequence were taken during 20 minutes with a variable duration scheme: 12 x 10s, 4 x10s, 4 x 30s, 3 x 300s. PET data acquisition was performed according to a protocol already described in the bibliography (Muzik O et al. J. Nucl. Med. 1993; 34:336-344). Once images were gathered for perfusion evaluation it was reserved 50 minutes to allow the physical decay of the ¹³N-ammonium radioactivity (semiperiod of desintegration 9,9 minutes). Subsequently, the acquisition of images was initiated for the metabolic trials of glucose, performed following the method of euglycaemic hyperinsulinaemic clamp (Knuuti MJ et al, J. Nucl. Med. 1992; 33:1255-1262). The ¹⁸F-FDG was injected by intravenous bolus after stabilizing glucose levels in a comprised range between 85 and 95 mg/dl 4,6 MBq/Kg, maximum 370 MBq. The serial image acquisition of ¹⁸F-FDG started in the instant of the injection and it continued for 60 minutes (8 x 15s, 2 x30s, 2 x 120s, 1 x 180s, 4 x 300s, 3 x 600s) following the protocol described by Knuuti et al mentioned supra.

Finished the images acquisition, it was performed a segmentation of the transmission, previous to the attenuation correction, the images of the metabolic trial were reconstructed using the OSEM method (ordered subsets expectation maximisation) with 2 iterations and 8 subsets. All the trials (as per perfusion as well as metabolism) were applied a gaussian filter (gaussian smoothering filter) of 6mm FWHM (full width at half maximum). For the usual analysis the transaxial images were reoriented according to the short axis, the vertical long axis and the horizontal long axis of the left ventriculus.

Finally, for the quantitative analysis there were used 6 contiguous sections of the medium region of the left ventriculus, in the short axis. The myocardial blood flow was calculated in absolute rates according to a 3 compartments model (Muzik O et al. J. Nucl. Med. 1993; 34:83-91) and the rates of glucose utilization (myocardial glucose utilization rates rMGU) were estimated by the Patlak graphical analysis (Patlak CS et al. J. Cereb. Blood Flow Metab. 1985;5:584-590).

### RESULTS

### 2.4 Cellular transplantation and postsurgical evolution

The demographical, clinical and functional features of the patients in the trial are gathered in the following tables 1 and 2.

**Table 1**

| **Features of patients** | | | | | | | |
|---|---|---|---|---|---|---|---|
| **Case** | **Age Sex** | **Local Evol.** | **Symptoms** | | **Class NYHA Local** | | |
| | | **Infarct** | **Infarct Angina/dyspneaBasal/FU** | | | | **Bypass** |
| | | **(months)** | | | | | |
| 1 | 63 M | Anterior | | 4 | Unstable/III | III/II | LAD, RC, |
| | OM | | | | | | |
| 2 | 64 M | Anterior-apical | 6 | III/II | II/I | LAD, | OM |
| 3 | 40 M | Inferior | | 5 | Unstable/II | II/I | RC, LAD, |
| 4 | 55 M | Anterior | | 23 | II/I | II/I | LAD, RC, |
| | OM | | | | | | |
| 5 | 68 M | Anterior-apical | 168 | III/III | III/II | LAD, RC, OM | |
| 6 | 74 F | Anterior | | 10 | /III | III/III | LAD |
| 7 | 69 M | Inferior | | 125 | Unstable/II | III/ | LAD, RC, |
| | OM, Dg | | | | | | |
| 8 | 71 M | Inferior | | 108 | Unstable/II | II/I | LAD, OM |
| 9 | 56 M | Anterior | | 120 | III/ | I/I | LAD, Dg, |
| | OM | | | | | | |
| 10 | 74 M | Inferior | | 20 | III/II | II/ | LAD, OM, |
| | Dg, RC | | | | | | |
| 11 | 68 M | Anterior | | 3 | I/I | I/I | LAD, RC, |
| | OM | | | | | | |
| 12 | 73 M | Anterior-apical | 146 | III/III | III/II | LAD, | Dg, OM |

### Abbreviations:

Sex. M: male; F: female
   Classification NYHA: Classification of the "New York Heart Association".
   FU: Evaluation of follow-up carried out 3 months after surgery and cellular transplantation.
Bypasses localization. LAD: left anterior descending artery;RC: right coronary artery; M: obtuse marginal artery; Dg: diagonal coronary artery.
During the operation of the aortocoronary bypass, once finished the grafting, and restored the spontaneous heart beat, the infarcted area was visualized and a solution between 3 to 5 ml was injected containing myogenic cells in a concentration up to 20 x 10⁶ cells/ml. The injection areas were identified for the echocardiographic follow-up. Before proceeding to the transplantation an immediately after the collection of cells for said transplantation, some microbiological cultures were carried out to prevent contamination. For this reason, the patient 9 was not made a transplantation, because his cell samples showed positive in the gram staining. Nevertheless, this patient was also made the protocolized trials for the follow-up.

**Table 2**

| **Features of patients** | | | | |
|---|---|---|---|---|
| **Case** | **Muscular** | **Myogenic cells** | **Basal LVEF LVEF FU** | |
| | **Biopsy(g)** | **implanted (x 10⁶)** | **2D/ABD** | **2D/ABD** |
| 1 | 10 | 318 | 35/37 | 55/56 |
| 2 | 13 | 165 | 40/45 | 50/53 |
| 3 | 7,5 | 192 | 45/46 | 70/65 |
| 4 | 7 | 393 | 40/47 | 62/66 |
| 5 | 10 | 200 | 27/26 | 40/50 |
| 6 | 9 | 110 | 30/38 | 40/48 |
| 7 | 9,5 | 171 | 40/38 | No FU |
| 8 | 5,5 | 105 | 40/42 | 47/51 |
| 9 | 9 | 0 | 45/40 | 35/38 |
| 10 | 14 | 390 | 25/29 | No FU |
| 11 | 10 | 100 | 40/43 | 51 /49 |
| 12 | 11,3 | 180 | 43/41 | 46/45 |

### Abbreviations:

LVEF. Left Ventricular Ejection Fraction
FU: Evaluation of follow-up carried out 3 months after surgery and cellular transplantation.
2D: bidimensional; ABD: automatic detection of the endocardial border.

The postsurgical evolution of the transplanted patients did not show complications. As in prior clinical trials it was observed cardiac arrythmias associated to the transplantation of skeletal myogenic cells, patients were monitorized during hospitalization (continuous telemetry) and at 40 days and 3 months of the transplantation (by a Holter-ECG, 24 hours). In any case it was observed a significant increase in arrythmias. What is more, the number of premature ventricular beats was reduced after transplantation. However, patient 6 developed non-sustained ventricular tachicardia 40 days after surgery. This patient undergone an aneurysmectomy during surgical procedure, what could explain the event.

The serologic analyses of cardiac and hepatic enzimes did not show significant changes after the transplantation and went progressively back to their normal rates. As indirect measure of inflammation, the protein c was determined, without observing drastic changes after the transplantation o during follow-up.

All patients have left hospital and are still alive.

### 2.5 Function of left ventriculus

The mean increase of the left ventriculus ejection fraction, measured by bidimensional echocardiography, was increased from 35,5±2,3% (mean ± standard mean error) before surgery up to 53,5±4,98% at 3 months of the transplantation (p<0,05). Global cardiac contractility, estimated by automatic echocardiography of endocardic border detection (ABD), increased from 39,8±3,26% to 56,3±3,1% (p<0,05) (Figure 1).

There was also an improvement in local contractility, shown by the decrease in the mean number of akinetic/hypokinetic/dyskinetic from 7(between 5-10, before surgery) to 3 (between 0-5, at 3 months of transplantation).

The regional basal motility index WMSI, average 1,72±0,14, was reduced at 3 months of the transplantation, 1,25±0,07 (p<0,05) (Table 3). To differentiate the potencial benefits over cardiac function which are derived from the transplantation of myogenic cells from those derived of the revascularization they compared the improvement in the WMSI indexes of the segments which received cell transplant and those of the segments which did not receive cells. The WMSI were drastically reduced at 3 months compared to basal indexes (before surgery), with a higher decrease in the segments that received cell transplantatation (Table 3).

The decrease of WMSI is linked to an improvement in the classification NYHA , from 2,2±0,2 basal to 1,5±0,26 at 3 months(p<0,01).

**Table 3**

| **Regional function (measured by the WMSI index, Wall motion score index)** | | | | |
|---|---|---|---|---|
| | **Basal** | **40 days** | **3 months** | **P** |
| Global | 1,73±0,07 | 1,40±0,07 | 1,25±0,07 | |
| 0,027 | | | | |
| Treated segments | 2,64±0,13 | 2,03±0,16 | 1,64±0,16 | |
| 0,027 | | | | |
| Non-treated segments | 1,29±0,13 | 1,1±0,06 | 1,05±0,04 | |
| 0,043 | | | | |

### 2.6 Miocardial perfusion and feasibility studies

Perfusion (PET-ammonium) and metabolic (FDG-glucose) studies were carried out in 7 patients by tests carried out before surgery (1 to 5 days earlier) and 3 months after transplantation. It was estimated for each region the tracer retention assigning each a quantitative numerical rate. The mean retention of glucose for all myocardium before surgery was 0,158±0,026 µmol.g-¹.min-¹ while at 3 months was 0,270±0,008 µmol.g-¹.min-¹ (p<0,05). In the differential analysis of the infarcted areas (necrotic tissue due to myocardial infarction) it was verified a significant increase of the retention of ammonnium and glucose, what suggests an improvement of the myocardial feasibility in the infarcted areas (Table 4). See also Figure 2.

**Table 4**

| **Positron emission tomography imaging PET. Evaluation of blood flow by ¹³N-ammonium retention (ml.g-¹.min-¹), and glucose metabolism by ¹⁸F-FDG (µmol.g-¹.min-¹).** | | | | | |
|---|---|---|---|---|---|
| | | | **Basal** | **3 months** | **P** |
| **Global** | | ¹⁸F-FDG | 0,158±0,026 | 0,270±0,008 | |
| | 0,028 | | | | |
| | | ¹³N-ammon | 0,47±0,05 | 0,5±0,003 | |
| | 0,273 | | | | |
| **Infarcted regions** | | ¹⁸F-FDG | 0,126±0,022 | 0,231±0,011 | |
| 0,028 | | | | | |
| | | ¹³N-ammon | 0,36±0,004 | 0,39±0,01 | |
| 0,686 | | | | | |
| **Non-infarcted reg** | | ¹⁸F-FDG | 0,170±0,029 | 0,284±0,013 | |
| 0,046 | | | | | |
| | | ¹³N-ammon | 0,59±0,007 | 0,62±0,06 | 0,5 |

### DISCUSSION

The main conclussions of this study are: 1. Direct transplantation of autologous skeletal myogenic cells to the myocardium of patients with history of myocardial infarction and with an operation of aortocoronary bypass is feasible and safe. 2. Although it cannot be clearly differentiated the derived effects of the bypass from the derived effects from the cellular transplantation, the studies of functionality and feasibility suggest that the transplantation of autologous myogenic cells contributes to improve contractility of left ventriculus and to the repair of tissues. 3. The skeletal myoblasts are able to be grafted, at least until 3 months after surgery.

Although to verify if skeletal myoblasts are able to be adequately grafted, it would be necessary to study directly the myocardium, the increase in glucose retention by PET shows clearly that in infarcted areas, where it was not detected viable tissue, it starts to be detected viable tissue once carried out the transplantation. On the other hand, although control patients were not included in the study, the results of ¹⁸F-FDG - PET for patient 9, who was only made the aortocoronary bypass by microbiological contamination of cell culture, did not show significant changes in glucose utilization at 3 months follow-up (Figure 2B), what without being conclusive, is encouraging.

These results show that aortocoronary bypass with transplantation of autologous skeletal myogenic cells improves remarkably cardiac contractility, made evident by the increase in the ejection fraction and particularly by the decrease in the WMSI index. From these results can not be concluded that improvement of the cardiac function is due only to the bypass. The fact that the myocardial segments which received cell transplantation have shown higher improvements, with an increase in the glucose retention in these same segments, shows that the improvement in the cardiac function can not be exclusively due to the bypass. For evidenced proof that the autologous cell transplantation is responsible for the improvement of the cardiac function it shall be necessary to carry out the transplantation without the cooperation of other therapies, something that nowadays would not be ethical unless the cells were implanted by percutaneous injection.

Recent researches about other clinical trials have suggested that the transplantation of autologous skeletal myoblasts is associated to cardiac arrythmias (Menasche P et al. Lancet 2001; 357:279-280. Menasche P et al. J. Am. Coll. Cardiol. 2003; 41:1078-1083. Hagege AA et al.Lancet 2003; 361:491-492. Pagani FD et al. J. Am. Coll. Cardiol. 2003; 41:879-888. In fact, some patients have required the implant of intracardiac defibrillators. Up to now, there is not known certainly the cause of these arrythmias, but they could be related to the formation of electrical reentry circuits (maybe because there are no gap junctions established with the implanted skeletal myoblasts), the number and volume of the implanted cells, or the use of the autologous myogenic cells. It is important to point out that in these protocols it has been used fetal bovine serum for the culture and expansion *in vitro* of the autologous myogenic cells, what constitutes a source of xenogenic proteins. The contact of the human cells with the bovine serum during 3 weeks bears fixation in the cell surface of said animal proteins. Transplantation of these cells would cause an inflammatory reaction followed by fibrosis. The clinical-pathological trials carried out show that the transplanted cells according to this method are embedded in a fibrotic pattern where there have not been neovascularization. This histological configuration represents a risk for the reentry circuits which can induce the ectopic generation of severe ventricular arrythmias. Unlike these researches, in the present clinical trial, there have been used expanded autologous skeletal myogenic cells in completely autologous culture mediums, avoiding any immune reaction. These could explain why in this clinical trial there have not been observed these arrythmias.

## Claims

1. An autologous culture medium of autologous human progenitor stem cells which comrpises:
a) between 0,1 % and 90% weight of autologous human serum;
b) between 0,1% and 10.000 UI/ml heparin;
c) between 0,1% and 10.000 UI/ml protamine; and
d) a culture medium with basic nutrients with or without glutamine, in sufficient quantity up to 100% weight.

2. A culture medium according to the Claim 1, in which said autologous human serum has been subject to treatments with the aim to inactivate the complement.

3. A culture medium according to the Claim 1, in which said autologous human serum has been obtained from blood samples of the patient.

4. A culture medium according to the Claim 1, in which said autologous human serum has been obtained by the realization of a plasmapheresis to the patient donor of said serum.

5. A culture medium according to the Claim 4, in which said plasmapheresis is carried out using heparin as anticoagulant and protamine sulphate to reverse anticoagulation.

6. A culture medium according to any of the previous Claims which comprises, furthermore, an antibiotic.

7. A culture medium according to Claim 6, in which said antibiotic is selected among penicillin, streptomycin, gentamicin and their mixtures.

8. A culture medium according to any of the previous Claims which comprises, furthermore, amphoterycin B, and/or a Fibroblast Growth Factor (FGF).

9. A culture medium according to Claim 1, which comprises:
89% medium HAM-F12;
10% autologous human serum of the patient
heparin 0,1 at 100UI/ml;
protamine 0,1 at 100UI/ml; y
1% penicillin/streptomycin and, optionally,
0,25mg/ml of amphoterycin B and/or
0,1 at 250 pg/ml of recombinant bFGF.

10. A method for the preparation of an autologous culture medium of autologous human progenitor stem cells, according to any of the Claims 1 to 9, which comprises the mixture of autologous human serum, heparin, protamine, basic nutrients with or without glutamine, together with, optionally, antibiotics, and/or amphoterycin B, and/or a Fibroblast Growth Factor.

11. A method according to Claim 10, in which said autologous human serum has been obtained by plasmapheresis.

12. Use of an autologous culture medium of autologous human progenitor stem cells, according to any of the Claims 1 to 9, for the culture *in vitro,* purification and expansion of autologous progenitor stem cells.

13. A method for the preparation of a composition of autologous human progenitor stem cells, which comprises the incubation of said autologous human progenitor stem cells in an autologous culture medium of autologous human progenitor stem cells, according to any of the Claims 1 to 9 and the purification of the autologous human progenitor stem cells obtained.

14. A method according to Claim 13, in which the purification of the autologous human progenitor stem cells obtained is carried out by the use of specific antibodies which allow the identification of extracellular antigens characteristic of the mentioned autologous human progenitor stem cells.

15. A method according to Claim 14, in which said specific and selective antibodies for the mentioned autologous human progenitor stem cells are joined to magnetic microspheres.

16. A method for the obtention of autologous human muscle progenitor stem cells, useful for their use in cellular therapy, which comprises to incubate said autologous human muscle progenitor stem cells in an autologous culture medium of autologous human progenitor stem cells according to any of the Claims 1 to 9, and purify the autologous human progenitor stem cells obtained.

17. A method according to Claim 16, in which the purification of autologous human muscle progenitor stem cells comprises the use of human anti-CD56 antibodies, optionally, joined to magnetic microspheres, and the selection of the cells which show a phenotype CD56+/ CD45-.

18. A method according to Claim 16, in which the purification of autologous human muscle progenitor stem cells comprises to subject cell culture to a stage of pre-plating with the aim to settle all or part of the fibroblasts present in said cell culture and, subsequently, to identify and separate the autologous human muscle progenitor stem cells by using human anti-CD56 antibodies, optionally, joined to magnetic microspheres, and the selection of the cells which show a phenotype CD56+/ CD45-.

19. A procedure for the obtention of autologous human muscle progenitor stem cells, from a biopsy of muscle tissue, for the preparation of a pharmaceutical composition, which comprises:
a)the realization of a biopsy in a patient object of the subsequent implant of the autologous human muscle progenitor stem cells to extract a fragment of skeletal tissue which comprises autologous human muscle progenitor stem cells;
b) the culture of said autologous human muscle progenitor stem cells from the skeletal muscle in an autologous culture medium of autologous progenitor stem cells according to any of the Claims 1 to 9, under conditions which allow the expansion of said cultured autologous human muscle progenitor stem cells;
c)the purification of said cultured autologous human muscle progenitor stem cells; and
d) the collection of said purified autologous human muscle progenitor stem cells; and optionally,
e) the freezing of said purified autologous human muscle progenitor stem cells until the preparation of said pharmaceutical composition.

20. A procedure according to Claim 19, which comprises the local administration to the patient, in the area of the biopsy, before the carrying out thereof, of a pharmaceutical composition which comprises a pharmacological agent which stimulates the proliferation of autologous human muscle progenitor stem cells.

21. A procedure according to Claim 19, in which said pharmacological agent comprises a local anesthetic, selected between lidocaine and bupivacaine.

22. A method according to Claim 19, in which the purification of autologous human muscle progenitor stem cells comprises to subject the cell culture to a stage of pre-plating with the aim to settle all or part of the fibroblasts present in said cell culture and, subsequently, to identify and separate the autologous human muscle progenitor stem cells by using human anti-CD56 antibodies, optionally, joined to magnetic microspheres, and the selection of the cells which show a phenotype CD56+/ CD45-.

23. Composition enriched in autologous human muscle progenitor stem cells which comprises, at least, 70% of the mentioned autologous human muscle progenitor stem cells.

24. Pharmaceutical composition which comprises, at least, 20 million cells, with a cell density of, at least, 50 million cells/ml and, at least, 40% of autologous progenitor stem cells CD56+/ CD45-, autologous culture medium of autologous progenitor stem cells according to any of the Claims 1 to 9 and, at least, one excipient acceptable from a pharmaceutical point of view.

25. Pharmaceutical composition according to Claim 24, which comprises between 20 and 200 million cells, with a cellular density between 50 and 70 million cells/ml, and at least, 70% autologous progenitor stem cells CD56+/ CD45-, autologous culture medium of autologous progenitor stem cells according to any of the Claims 1 to 9 and human albumin in a quantity between 0,1% and 20% eight with respect to the total quantity.

26. A therapeutical procedure of autologous cellular cardiomyoplasty to create, regenerate and repair dysfunctional myocardial tissue by means of the implant of a pharmaceutical composition which comprises autologous human muscle progenitor stem cells, regenerators of cardiac tissue, and *ex vivo* expanded in an autologous culture medium, comprising said procedure the collection of a material sample from the body of the patient object of the subsequent implant which includes autologous human muscle progenitor stem cells, the expansion of the mentioned cells by culture in an autologous culture medium of autologous progenitor stem cells according to any of the Claims 1 to 9, and the implantation of the collected autologous human progenitor stem cells in the patient, who previously had been extracted such material containing the autologous human muscle progenitor stem cells.

27. A therapeutical procedure of autologous cellular cardiomyoplasty to create, regenerate and repair dysfunctional myocardial tissue by means of the implant of a pharmaceutical composition which comprises autologous human muscle progenitor stem cells, regenerators of cardiac tissue, and ex *vivo* expanded in an autologous culture medium; and where said procedure comprises the following steps:
a) the collection from the patient of an skeletal muscle biopsy taken from a muscle, preferably, preconditioned by an intramuscular injection of a local anesthetic;
b) the preparation of a culture medium of autologous human progenitor stem cells, according to any of the Claims 1 to 9, from the autologous serum of the patient;
c) the preparation of a composition enriched in autologous human muscle progenitor stem cells from the biopsy of a) and the culture medium of b);
d) the preparation of a pharmaceutical composition from the composition of c); and
e) the implant of a pharmaceutical composition of autologous human progenitor stem cells of d) in myocardial lesions.

28. A procedure according to Claim 27, in which the implant of said composition of autologous human progenitor stem cells is carried out by direct injection in the peripheral region to the infarction, scar or by injection in the intracoronary spaces of both ventriculi.

29. A procedure according to Claim 27, in which the implant of said composition of autologous human progenitor stem cells is carried out by systemic or intracoronary administration by percutaneous venous access.

30. A procedure according to Claim 27, in which the implant of said composition of autologous human progenitor stem cells is carried out by a robotized and computerized system.
